# FASCICULE DE BREVET EUROPEEN

(11) **EP 0 882 698 B1**
(45) Date de publication et mention de la délivrance du brevet: **14.08.2002**
(21) Numéro de dépôt: 98401203.9
(22) Date de dépôt: 19.05.1998
(51) Int. Cl.: C07C 45/50, C07C 47/02

(54) **Procédé pour l'hydroformylation d'olefines**
Verfahren zur Hydroformylierung von Olefininen
Process for the hydroformylation of olefins

(30) Priorité: 27.05.1997 FR 9706570
(43) Date de publication de la demande: 09.12.1998
(73) Titulaire: INSTITUT FRANCAIS DU PETROLE, 92852 Rueil-Malmaison Cedex (FR)
(72) Inventeur: Olivier, Hélène, 92500 Rueil Malmaison (FR); Commereuc, Dominique, 92190 Meudon (FR); Drochon, Sébastien, 92500 Rueil Malmaison (FR)

(56) Documents cités:
- EP-A- 0 107 430
- EP-A- 0 776 880
- US-A- 3 832 391
- MIYAZAWA C ET AL: "HYDROFORMYLATION OF OLEFINS IN THE PRESENCE OF RHODIUM AND A PHOSPHINE OXIDE" CHEMICAL ABSTRACTS + INDEXES, vol. 111, no. 3, 17 juillet 1989, page 574 XP000017118 & JP 63 222 139 A (MITSUBISHI KASEI CORP.) 16 septembre 1988

## Description

L'objet de la présente invention est un nouveau procédé d'hydroformylation d'oléfines et en particulier d'oléfines internes par de l'oxyde de carbone et de l'hydrogène dans lequel le système catalytique est une solution d'au moins un composé d'un métal de transition dans un sel ionique non aqueux organique-inorganique liquide à la température de réaction, et dans lequel les produits issus de la réaction d'hydroformylation sont peu ou pas solubles.

L'hydroformylation des composés oléfiniques est une réaction de grande importance industrielle et la plupart des procédés ont recours à des catalyseurs qui sont dissous dans une phase organique constituée des réactifs, des produits et éventuellement d'un excès de ligands, si bien que l'on rencontre des difficultés pour séparer et récupérer le catalyseur, en particulier quand celui-ci est un métal noble comme par exemple le rhodium.

Une solution en vue de résoudre ce problème a été décrite dans le brevet français FR-2.314.910. Elle consiste à effectuer l'hydroformylation en présence d'une solution aqueuse contenant un complexe du rhodium qui est rendu hydrosoluble grâce à la présence d'un ligand phosphine sulfonée lui-même hydrosoluble tel que le sel de sodium de la triphénylphosphine trisulfonée. De cette manière la phase organique contenant les aldéhydes est aisément séparée de la phase aqueuse contenant le catalyseur. Cette technique a fait l'objet d'un nombre considérable de travaux qui ont été discutés dans un article de W.A. Herrmann paru dans Angewandte Chemie International en 1993, volume 32, page 1524 et suivantes. En dépit du grand intérêt industriel de cette technique pour l'hydroformylation du propylène, ce système à deux phases souffre du manque de solubilité des oléfines dans l'eau, ce qui conduit à des vitesses de réaction relativement faibles qui la rend inapplicable pour les oléfines à longue chaîne.

Par ailleurs a été décrit dans le brevet U.S. 3.565.823 une technique consistant à disperser un composé d'un métal de transition dans un sel d'étain ou de germanium et d'un ammonium ou d'un phosphonium quaternaire, de formule (R₁R₂R₃R₄Z)YX₃ dans laquelle R₁,R₂,R₃,R₄, sont des restes hydrocarbyles ayant jusqu'à 18 atomes de carbone, Z est l'azote ou le phosphore, Y l'étain ou le germanium et X un halogène, chlore ou brome, ce milieu non-aqueux à caractère ionique constituant un "sel fondu". Dans le brevet U.S. 3.657.368 a été décrit un procédé d'hydrogénation des oléfines et dans le brevet U.S. 3.919.271 un procédé d'hydrogénation des nitriles utilisant tous deux la composition précédente à base d'étain et de germanium. Dans le brevet U.S. 3.832.391 a été revendiqué un procédé de carbonylation des oléfines par la même composition.

Les compositions précédemment décrites présentent le désavantage d'avoir un point de fusion relativement élevé, la réaction d'hydroformylation ayant alors lieu par exemple à au moins 90°C.

Il a été proposé dans la demande de brevet française N°95/14147 du 30 novembre 1995, correspondant à EP-A-0 776 880 que l'on pouvait à la fois bénéficier des avantages d'une mise en oeuvre à deux phases tout en évitant les inconvénients liés d'une part à l'utilisation de l'eau et d'autre part à l'emploi des composés à point de fusion élevé, en dissolvant les composés catalytiques de métaux de transition des groupes 8, 9 et 10 et en particulier les composés du cobalt, du ruthénium, du rhodium, de l'iridium, du palladium et du platine, connus pour catalyser l'hydroformylation, dans des sels organiques-inorganiques liquides à basse température.

Il a maintenant été trouvé que l'addition d'oxyde de phosphine à la composition catalytique précédente permettait de réaliser l'hydroformylation des oléfines et en particulier des oléfines internes avec de bonnes activités et sélectivités en aldéhydes.

Plus précisément, l'invention a pour objet un procédé pour l'hydroformylation en phase liquide d'oléfines (ou composés oléfiquement insaturés) et en particulier d'oléfines internes, procédé dans lequel la réaction est effectuée en présence d'au moins une oxyde de phosphine et d'un sel organique-inorganique de formule générale Q⁺A⁻, dans laquelle Q⁺ représente un ammonium quaternaire et/ou phosphonium quaternaire, et A⁻ représente un anion, ledit sel ne contenant pas d'étain ou de germanium, et d'au moins un composé d'un métal de transition (des groupes 8, 9 et 10).

Les sels liquides selon l'invention ont pour formule générale Q⁺ A⁻ dans laquelle Q⁺ représente un ammonium quaternaire et/ou d'un phosphonium quaternaire et A⁻ représente tout anion connu comme étant non-coordinant susceptible de former un sel liquide à basse température, c'est-à-dire en dessous de 150°C et avantageusement en-dessous de 90°C, et de préférence d'au plus 85° C, et de préférence d'au plus 50°C tels de préférence les ions tétrafluoroborate, hexafluorophosphate, hexafluoroantimonate, hexafluoroarsénate, trifluorométhylsulfonate, fluorosulfonate. On peut aussi utiliser les anions dichlorocuprate, tétrachloroborate, tétrachloroaluminate, trichlorozincate mais de façon non préférée. On préfère exclure les halogénures. Les ammonium et/ou phosphonium quaternaires répondent de préférence aux formules générales NR¹R²R³R⁴⁺ et PR¹R²R³R⁴⁺, ou aux formules générales R¹R²N=CR³R⁴⁺ et R¹R²P=CR³R⁴⁺ où R¹, R², R³ et R⁴, identiques ou différents, représentent l'hydrogène à l'exception du cation NH₄^{⁺} et de préférence un seul substituant peut représenter l'hydrogène, ou des restes hydrocarbyles ayant de 1 à 12 atomes de carbone, par exemple des groupements alkyles, saturés ou non saturés, cycloalkyles ou aromatiques, aryle ou aralkyle, comprenant de 1 à 12 atomes de carbone. Les ammonium et/ou phosphonium peuvent également être dérivés d'hétérocycles azotés ou phosphorés comportant 1, 2 ou 3 atomes d'azote et/ou de phosphore, de formule générales: 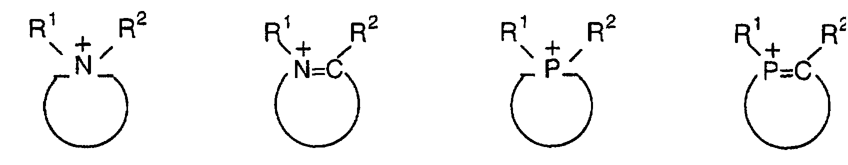 dans lesquelles les cycles sont constitués de 4 à 10 atomes, de préférence 5 à 6 atomes, R¹ et R² étant définis comme précédemment. L'ammonium ou le phosphonium quaternaire peuvent également être un cation de formule:

R¹R²⁺N=CR³-R⁵-R³C=N⁺R¹R²

R¹R²+P=CR³-R⁵-R³C=P⁺R¹R²

dans laquelle R¹,R²,R³, identiques ou différents sont définis comme précédemment et R⁵ représente un reste alkylène ou phénylène. Parmi les groupements R¹, R², R³ et R⁴ on mentionnera les radicaux méthyl, éthyl, propyl, isopropyl, butyl, secondaire butyl, tertiaire butyl, amyl, méthylène, éthylidène, phényl ou benzyl; R⁵ pourra être un groupement méthylène, éthylène, propylène ou phénylène. Le cation ammonium et/ou phosphonium est choisi de préférence dans le groupe formé par le N-butylpyridinium, le N-éthylpyridinium, le butyl-3 méthyl-1 imidazolium, le diéthylpyrazolium, l'éthyl-3 méthyl-1 imidazolium, le pyridinium, le triméthylphénylammonium, le tétrabutylphosphonium. A titre d'exemples des sels utilisables selon l'invention on peut citer l'hexafluorophosphate de N-butylpyridinium, le tétrafluoroborate de N-éthyl pyridinium, le tétrafluoroborate de tétrabutylphosphonium, l'hexafluoroantimonate de butyl-3 méthyl-1 imidazolium, l'hexafluorophosphate de butyl-3 méthyl-1 imidazolium, le trifluorométhylsulfonate de butyl-3 méthyl-1 imidazolium, le fluorosulfonate de pyridinium, l'hexafluorophosphate de triméthylphénylammonium. Ces sels peuvent être utilisés seuls ou en mélange. Ils ont une fonction de solvant. Peuvent être également utilisés, par exemple, le dichlorocuprate de butyl-3-méthyl-1-imidazolium, le tétrachloroborate de pyridinium, le tétrachloroaluminate de butyl-3-méthyl-1-imidazolium, le trichlorozincate de butyl-3-méthyl-1-imidazolium.

Les composés des métaux de transition utilisables selon l'invention sont de façon générale tous les composés des métaux de transition des groupes 8, 9 et 10 et notamment ceux connus de l'homme de l'art pour hydroformyler les oléfines. Ils peuvent être utilisés seuls ou en mélange. Ils peuvent être complexés ou associés à un ligand organique. Ils peuvent être mis en oeuvre sous forme de sels, et de façon préférée, qui n'est pas un halogénure. Il s'agit entre autres des composés du cobalt, du rhodium, de l'iridium, du ruthénium, du palladium et du platine. Le choix du composé catalytique du métal de transition n'est pas critique. On mentionnera, par exemple, HRh(CO)(PR₃)₃, HRh(CO)₂(PR₃), HRh(CO) [P(OR)₃]₃, Rh(acac)(CO)₂, (acac signifiant acétylacétonate) Rh₆(CO)₁₆, [Rh(norbornadiène)(PPh₃)₂]⁺[PF₆]⁻, [Rh(CO)₃(PPh₃)₂]⁺[BPh₄]⁻, RhCI (CO)(PEt₃)₂, [RhCl(cyclooctadiène)]₂, [Rh(CO)₃(PR₃)₂]⁺BPh₄⁻, [Rh(CO)₃ (PR₃)₂]⁺PF₆⁻, HCo(CO)₄, Ru₃(CO)₁₂, [RuH(CO) (acétonitrile)₂(PPh₃)₃] ⁺[BF₄]⁻, PtCl₂(cyclooctadiène), [Ir(CO)₃(PPh₃)]⁺[PF₆]⁻, [HPt(PEt₃)₃]⁺[PF₆].⁻ Mais on peut aussi avoir recours à des sels totalement inorganiques, précurseurs de catalyseur, tels que Rh₂O₃, Pd(NO₃)₂ et Rh(NO₃)₃, et de façon non préférée à des halogénures tel que RhCl₃,3H₂O.
Les oxydes de phosphine selon l'invention ont pour formule générale R₁R₂R₃PO, dans laquelle R₁, R₂, R₃, identiques ou différents représentent des groupes hydrocarbyles ayant de préférence de 1 à 12 atomes de carbone, par exemple des groupements alkyles, saturés ou non saturés, cycloalkyles ou aromatiques, aryle ou aralkyle, comprenant de 1 à 12 atomes de carbone. Ces ligands peuvent être mono ou bidentés. Ils peuvent porter, en outre une autre fonction telle que amine, ammonium, alcool, acide carboxylique, sulfonate.
La composition catalytique est obtenue par mélange, d'une manière quelconque, du sel liquide avec le composé du métal de transition et éventuellement le ligand.
Les complexes catalytiques métal de transition-ligand organique peuvent être préparés en dehors du milieu réactionnel et y être introduits pour la réaction. Ils peuvent également être formés in situ, dans le milieu réactionnel, par introduction des composants nécessaires à leur formation.
Un autre avantage lié au procédé de la présente invention réside en ce que on peut uliliser une très grande variété de ligands qui ne sont pas compatibles avec l'eau mais qui sont stables dans ces milieux, comme par exemple les phosphites qui sont très facilement hydrolysables et dont la synthèse est bien plus aisée que celle des phosphines.

D'une façon générale, la composition catalytique peut contenir un solvant organique miscible ou partiellement miscible comme un hydrocarbure aromatique, et/ou un hydrocarbure aliphatique non miscible qui permet une meilleure séparation des phases. De façon préférée, la composition catalytique ne contient pas d'eau.

La concentration du composé (du complexe, de préférence) de métal de transition dans le "sel fondu" n'est pas critique. Elle est avantageusement comprise entre 1 mmole de composé par litre de "sel fondu" et 500 mmoles par litre, de préférence entre 2 et 200 mmoles par litre, et encore entre 2 et 100, voire 2 à 50. Le rapport molaire entre le ligand organique et le composé du métal de transition peut être compris entre 1 et 100, de préférence entre 1 et 20.

Les composants entrant dans la composition selon l'invention, peuvent être mélangés dans un ordre quelconque, à une température comprise entre -20°C et + 200°C, et de préférence 30°C à moins de 150°C et avantageusement de 0°C à moins de 150°C, 0°C à 120°C, ou encore 0 - moins de 90°C, de préférence 0 - 85°C ou 0 à 50°C.

Les composés oléfiniquement insaturés susceptibles d'être hydroformylés selon l'invention sont les monooléfines internes. A titre d'exemple on peut citer l'hydroformylation du butène-2 en pentanal et isopentanals, des pentènes en hexanal et isohexanals, des hexènes en isoheptanals, des isooctènes en isononanals. Ces composés peuvent être utilisés purs ou dilués par des hydrocarbures saturés ou insaturés.

Le rapport des pressions partielles du monoxyde de carbone a l'hydrogène utilisé dans le milieu réactionnel pour l'hydroformylation peut être de 1:10 à 10:1, de préférence dans un rapport 1:1, mais tout autre rapport peut être utilisé selon la mise en oeuvre du procédé.

La température à laquelle se fera l'hydroformylation sera comprise entre 30°C et 200°C, avantageusement la température est inférieure à 150°C, de préférence entre 50°C et moins de 150°C de préférence inférieure à 90°C, et encore plus avantageusement d'au plus 85°C. Une gamme de températures préférées est entre 50°C et moins de 150°C et encore plus avantageusement de 30°C à 120°C, de 30°C à moins de 90°C. La pression peut être comprise entre 1 MPa et 20 MPa, de préférence entre la 2MPa et 10 MPa.

La réaction catalytique d'hydroformylation des oléfines internes peut être conduite en système fermé, en système semi-ouvert ou en continu avec un ou plusieurs étages de réaction. A la sortie du réacteur la phase organique contenant les produits de réaction (aldéhydes) est séparée avantageusement par simple décantation de la phase polaire catalytique contenant le "sel fondu" et la majeure partie du catalyseur. La phase polaire qui contient au moins en partie le catalyseur, est, au moins en partie, retournée au réacteur, l'autre partie étant traitée pour éliminer les résidus du catalyseur.

Les exemples suivants illustrent l'invention sans en limiter la portée:

### EXEMPLE 1

Dans un réacteur en acier inoxydable à double enveloppe d'une contenance de 100 mL purgé de l'air et de l'humidité et placé sous la pression atmosphérique du mélange hydrogène-oxyde de carbone, on a introduit 4 mL d'hexafluorophosphate de butylméthylimidazolium, 19,3 mg (0.075 mmole) du complexe Rh(acétylacétonate)(CO)₂ et 197 mg (0,71 mmole) d'oxyde de triphénylphosphine dissous dans 2 mL de toluène, 2 mL d'heptane (étalon) et 7,5 mL (68 mmole) de 2-pentène. On a porté la pression du mélange hydrogène-oxyde de carbone à 5 MPa et la température à 80°C et on a mis l'agitation en route. Après 2 heures on a arrêté l'agitation et on a laissé se décanter le mélange; on a soutiré la phase organique surnageante qui était très légèrement colorée. La conversion du 2-pentène était de plus de 80 %. Le rendement molaire était de 17% en hexanal et de 65% en 2-méthylpentanal. Le reste était constitué de 2-pentène et de traces de 1-pentènes (<1%).

### EXEMPLE 2 (comparatif)

On a opéré dans l'appareillage décrit dans l'exemple 1. On y a introduit 19,3 mg (0.075 mmole) du complexe Rh(acétylacétonate)(CO)₂ et 197 mg (0,71 mmole) d'oxyde de triphénylphosphine dissous dans 7,5 mL de toluène, 20 mL d'heptane et 7,5 mL (68 mmole) de 2-pentène. On a porté la pression du mélange hydrogène-oxyde de carbone à 5 MPa et la température à 80°C et on a mis l'agitation en route. Après 2 heures on a arrêté l'agitation, dégazé et soutiré la phase organique. La conversion du 2-pentène était de 30% et la sélectivité en aldéhydes de 99%, le reste étant du 1-pentène.

## Revendications

1. Procédé d'hydroformylation en phase liquide d'au moins une oléfine par de l'oxyde de carbone et de l'hydrogène en présence d'une composition catalytique contenant au moins un composé d'un métal de transition, au moins un sel organique inorganique ne contenant pas d'étain ou de germanium, ledit sel est un sel d'ammonium et/ou phosphonium quaternaire de formule générale Q⁺A⁻ dans laquelle Q⁺ représente un ammonium et/ou un phosphonium quaternaire, et A⁻ représente un anion, et au moins un oxyde de phosphine de formle R₁R₂R₃ PO, avec R₁,R₂,R₃ identiques ou différents représentent des groupes hydrocarbyles choisis dans le groupe formé par les groupements alkyles saturés ou non saturés, cycloalkyles, aromatiques, aryle ou alkylaryle, à l'exception de l'oxyde de triphénylphosphine, et ayant de 1 à 12 atomes de carbone.

2. Procédé selon la revendication 1 dans lequel l'oléfine est uns oléfine interne.

3. Procédé selon l'une des revendications 1 ou 2 dans lequel l'anion A⁻ est choisi dans le groupe formé par le tétrafluoroborate, l'hexafluorophosphate, l'hexafluoroantimonate, l'hexafluoroarsénate, le trifluorométhylsulfonate, le fluorosulfonate, ou bien encore le dichlorocuprate, le tétrachloroborate, le tétrachloroaluminate, le trichlorozincate.

4. Procédé selon l'une des revendications 1 à 3 dans lequel le cation ammonium et/ou phosphonium quaternaires sont choisis dans le groupe formé par les cations de formule générale:
R¹R²R³R⁴N⁺
R¹R²N=CR³R⁴⁺
R¹R²R³R⁴P⁺
R¹R²P=CR³R⁴⁺ 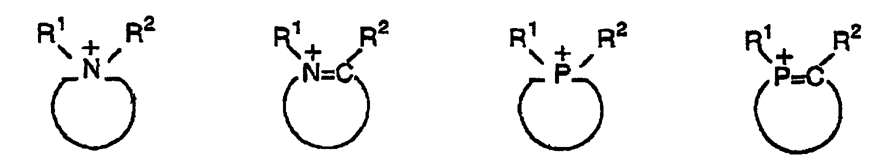
dans lesquelles R¹,R²,R³,R⁴, identiques ou différents représentent l'hydrogène, à l'exception de NH₄^{⁺}, et des restes hydrocarbyles ayant 1 à 12 atomes de carbone, et dans lesquelles les cycles sont constitués de 4 à 10 atomes.

5. Procédé selon l'une des revendications 1 à 3 dans laquelle le cation ammonium et/ou phosphonium quaternaires ont pour formules générales:
R¹R²⁺N=CR³-R⁵-R³C=N⁺R¹R²
R¹R²⁺P=CR³-R⁵-R³C=P⁺R¹R²
dans laquelle R¹,R²,R³ identiques ou différents représentent l'hydrogène ou des restes hydrocarbyles ayant 1 à 12 atomes de carbone, et R⁵ représente un reste alkylène ou phénylène.

6. Procédé selon l'une des revendications précédentes dans lequel le cation ammonium et/ou phosphonium quaternaires sont choisis dans le groupe constitué par le N-butylpyridinium, le N-éthylpyridinium, le butyl-3 méthyl-1 imidazolium, le diéthylpyrazolium, l'éthyl-3 méthyl-1 imidazolium, le pyridinium, le triméthylphénylammonium, le tétrabutylphosphonium.

7. Procédé selon l'une des revendications précédentes dans lequel les sels d'ammonium et/ou de phosphonium quaternaires sont choisis dans le groupe constitué par l'hexafluorophosphate de N-butylpyridinium, le tétrafluoroborate de N-éthyl pyridinium, le tétrafluoroborate de tétrabutylphosphonium, l'hexafluoroantimonate de butyl-3 méthyl-1 imidazolium, l'hexafluorophosphate de butyl-3 méthyl-1 imidazolium, le trifluoro-méthylsulfonate de butyl-3 méthyl-1 imidazolium, le fluorosulfonate de pyridinium, l'hexafluorophosphate de triméthylphénylammonium, le dichlorocuprate de butyl-3 méthyl-1 imidazolium, le tétrachloroborate de pyridinium, le tétrachloroaluminate de butyl-3 méthyl-1 imidazolium, le trichlorozincate de butyl-3 méthyl-1 imidazolium.

8. Procédé selon l'une des revendications précédentes dans lequel le métal de transition est le cobalt, le rhodium, l'iridium, le ruthénium, le palladium et le platine.

9. Procédé selon les revendications précédentes dans lequel le composé de métal de transition est un complexe de métal de transition.

10. Procédé selon l'une des revendications précédentes dans lequel le composé du métal de transition est choisi dans le groupe formé par HRh(CO)(PR₃)₃, HRh(CO)₂(PR₃), HRh(CO)[P(OR)₃]₃, Rh(acac)(CO)₂, (acac signifiant acétylacétonate) Rh₆(CO)₁₆, [Rh(CO)₃(PPh₃)₂]⁺[BPh₄]⁻, RhCl(CO)(PEt₃)₂, [RhCl(cyclooctadiène)]₂, [Rh(CO)₃(PR₃)₂]⁺BPh₄⁻, [Rh(CO)₃(PR₃)₂]⁺PF₆⁻, [Rh(norbornadiène)(PPh₃)₂]⁺[PF₆]⁻, HCo(CO)₄, Ru₃(CO)₁₂, [RuH(CO)(acétonltrile)₂(PPh₃)₃]⁺[BF₄]⁻, PtCl₂(cyclooctadiène), [Ir(CO)₃(PPh₃)]⁺[PF₆]⁻, [HPt(PEt₃)₃]⁺[PF₆]⁻, Rh₂O₃, Pd(NO₃)₂ et Rh(NO₃)₃,

11. Procédé selon l'une des revendications 1 à 10 dans lequel l'oxyde de phosphine contient au moins une fonction aminé, ammonium, alcool, acide carboxylique ou sulfonate.

12. Procédé selon l'une des revendications précédentes dans lequel la concentration du ou des composés du ou des métaux de transition par rapport au sel d'ammonium et/ou de phosphonium est de 1 à 500 mmoles par litre.

13. Procédé selon l'une des revendications précédentes dans lequel la composition catalytique contient également un solvant organique.

14. Procédé selon la revendication 13 dans lequel le solvant est choisi dans le groupe formé par les hydrocarbures aromatiques et les hydrocarbures aliphatiques.

15. Procédé selon l'une des revendications 1 à 14 dans lequel la phase organique contenant les aldéhydes est séparée de la phase polaire, ladite phase polaire contenant au moins une partie du catalyseur étant au moins en partie recyclée dans le réacteur d'hydroformylation.

16. Procédé selon l'une des revendications 1 à 15 opérant entre 30 et 200°C, sous une pression totale comprise entre 1 MPa et 20 MPa, les rapports des pressions partielles de l'oxyde de carbone à l'hydrogène étant de 1:10 à 10:1.

## Claims

1. Process for liquid-phase hydroformylation of at least one olefin by carbon oxide and hydrogen in the presence of a catalytic composition that contains at least one compound of a transition metal, at least one organic-inorganic salt that does not contain tin or germanium; said salt is a quaternary ammonium salt and/or a quaternary phosphonium salt of general formula Q⁺A⁻, in which Q⁺ represents a quaternary ammonium and/or a quaternary phosphonium, and A⁻ represents an anion, and at least one phosphine oxide of general formula R¹ R² R³ PO, where R¹, R², R³, which are identical or different, represent hydrocarbonyl radicals that are selected from the group consisting of saturated or unsaturated alkyl groups, cycloalkyl, aromatic, aryl or arylalkyl except triphenylphosphine oxide and that have from 1 to 12 carbon atoms.

2. Process according to claim 1, wherein the olefin is an internal olefin.

3. Process according to one of claims 1 or 2, wherein anion A⁻ is selected from the group that is formed by tetrafluoroborate, hexafluorophosphate, hexafluoroantimonate, hexafluoroarsenate, trifluoromethylsulfonate, fluorosulfonate, or else dichlorocuprate, tetrachloroborate, tetrachloroaluminate, or trichlorozincate.

4. Process according to one of claims 1 to 3, wherein the quaternary ammonium cation and/or quaternary phosphonium cation are selected from the group that is formed by the cations of general formula:
R¹R²R³R⁴N⁺
R¹R²N=CR³R⁴⁺
R¹R²R³R⁴P⁺
R¹R²P=CR³R⁴⁺ 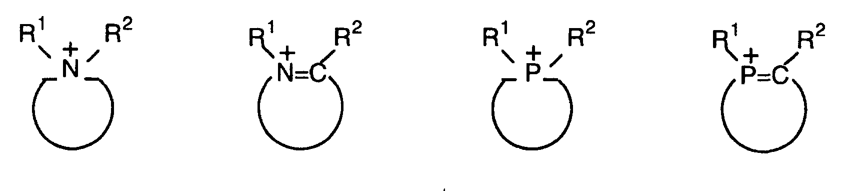
wherein R¹, R², R³, and R⁴, which are identical or different, represent hydrogen, with the exception of NH₄⁺, and hydrocarbyl radicals that have 1 to 12 carbon atoms, and wherein the cycles consist of 4 to 10 atoms.

5. Process according to one of claims 1 to 3, wherein the quaternary ammonium cation and/or quaternary phosphonium cation have for general formulas:
R¹R²⁺N=CR³-R⁵-R³C=N⁺R¹R²
R¹R²⁺P=CR³-R⁵-R³C=P⁺R¹R²
in which R¹, R², and R³, which are identical or different, represent hydrogen or hydrocarbyl radicals that have 1 to 12 carbon atoms, and R⁵ represents an alkylene or phenylene radical.

6. Process according to one of the preceding claims, wherein the quaternary ammonium cation and/or quaternary phosphonium cation are selected from the group that consists of N-butylpyridinium, N-ethylpyridinium, butyl-3 methyl-1 imidazolium, diethylpyrazolium, ethyl-3 methyl-1 imidazolium, pyridinium, trimethylphenylammonium, and tetrabutylphosphonium.

7. Process according to one of the preceding claims, wherein the quaternary ammonium salts and/or quaternary phosphonium salts are selected from the group that consists of N-butylpyridinium hexafluorophosphate, N-ethyl pyridinium tetrafluoroborate, tetrabutylphosphonium tetrafluoroborate, butyl-3 methyl-1 imidazolium hexafluoroantimonate, butyl-3 methyl-1 imidazolium hexafluorophosphate, butyl-3 methyl-1 imidazolium trifluoromethylsulfonate, pyridinium fluorosulfonate, trimethylphenylammonium hexafluorophosphate, butyl-3 methyl-1 imidazolium dichlorocuprate, pyridinium tetrachloroborate, butyl-3 methyl-1 imidazolium tetrachloroaluminate, and butyl-3 methyl-1 imidazolium trichlorozincate.

8. Process according to one of the preceding claims, wherein the transition metal is cobalt, rhodium, iridium, ruthenium, palladium, and platinum.

9. Process according to the preceding claims, wherein the transition metal compound is a transition metal complex.

10. Process according to one of the preceding claims, wherein the compound of the transition metal is selected from the group that is formed by HRh(CO)(PR₃)₃, HRh(CO)₂(PR₃), HRh(CO)-[P(OR)₃]₃, Rh(acac)(CO)₂ (where acac means acetylacetonate), Rh₆(CO)₁₆, [Rh(CO)₃(PPh₃)₂]⁺[BPh₄]⁻, RhCl(CO)(PEt₃)₂, [RhCl(cyclooctadiene)]₂, [Rh(CO)₃(PR₃)₂]⁺BPh₄-, [Rh(CO)₃(PR₃)₂]⁺PF₆⁻, [Rh(norbornadiene)(PPh₃)₂]⁺[PF₆]-, HCo(CO)₄, Ru₃(CO)₁₂, [RuH(CO) (acetonitrile)₂(PPh₃)₃]⁺[BF₄]⁻, PtCl₂(cyclooctadiene), [Ir(CO)₃(PPH₃)]⁺[PF₆]-, [HPt(PEt₃)₃]⁺[PF₆]-, RH₂O₃, Pd(NO₃)₂ and Rh(NO₃)₃.

11. Process according to one of claims 1 to 10, wherein the phosphine oxide contains at least one amine, ammonium, alcohol, carboxylic-acid, or sulfonate group.

12. Process according to one of the preceding claims, wherein the concentration of the compound or compounds of the transition metal or transition metals relative to the ammonium salt and/or phosphonium salt is 1 to 500 mmol per liter.

13. Process according to one of the preceding claims, wherein the catalytic composition also contains an organic solvent.

14. Process according to claim 13, wherein the solvent is selected from the group that is formed by the aromatic hydrocarbons and the aliphatic hydrocarbons.

15. Process according to one of claims 1 to 14, wherein the organic phase that contains the aldehydes is separated from the polar phase, whereby said polar phase contains at least a portion of the catalyst that is at least partly recycled into the hydroformylation reactor.

16. Process according to one of claims 1 to 15 that operates between 30 and 200°C, under a total pressure of between 1 MPa and 20 MPa, whereby the partial-pressure ratios of carbon oxide to hydrogen are 1:10 to 10:1.

## Patentansprüche

1. Verfahren zur Hydroformylierung in flüssiger Phase von wenigstens einem Olefin mit Kohlendioxid und Wasserstoff in Gegenwart einer katalytischen Zusammensetzung, die enthält: wenigstens eine Verbindung eines Übergangsmetalls, wenigstens ein organisch-anorganisches Salz, das nicht Zinn oder Germanium enthält, wobei das Salz ein quartemäres Ammoniumoder quarternäres Phosphoniumsalz der allgemeinen Formel Q⁺A⁻ ist, in dem Q⁺ ein quarternäres Ammonium- oder quarternäres Phosphonium darstellt, und A⁻ ein Anion darstellt, und wenigstens ein Phosphinoxid der Formel R₁R₂R₃PO mit identischen oder verschiedenen R₁R₂R₃, die Hydrocarbylgruppen darstellen, welche aus der Gruppe gewählt sind, die gebildet wird durch die gesättigten oder nicht-gesättigten Alkyl-, Cycloalkyl-, Aromaten-, Aryl- oder Alkylarylgruppen, mit Ausnahme des Oxids von Triphenylphosphin, und welche 1 bis 12 Kohlenstoffatome haben.

2. Verfahren nach Anspruch 1, **dadurch gekennzeichnet, dass** das Olefin ein internes Olefin ist.

3. Verfahren nach Anspruch 1 oder 2, **dadurch gekennzeichnet, dass** das Anion A⁻ aus der Gruppe gewählt wird, die gebildet wird durch Tetrafluorborat, Hexafluorphosphat, Hexafluorantimonat, Hexafluorarsenat, Trifluorethylsulfonat, Flursulfonat, oder auch Dichlorcuprat, Tetrachlorbrorat, Tetrachloraluminat, Trichlorzinkat.

4. Verfahren nach einem der Ansprüche 1 bis 3, **dadurch gekennzeichnet, dass** die quarternären Ammonium- und/oder Phosphoniumkationen aus der Gruppe gewählt werden, die gebildet wird durch die Kationen mit allgemeiner Formel:
**R**^{**1**}**R**^{**2**}**R**^{**3**}**R**^{**4**}**N**^{**+**}
**R**^{**1**}**R**^{**2**}**N=CR**^{**3**}**R**^{**4+**}
**R**^{**1**}**R**^{**2**}**R**^{**3**}**R**^{**4**}**P**^{**+**}
**R**^{**1**}**R**^{**2**}**P=CR**^{**3**}**R**^{**4+**} 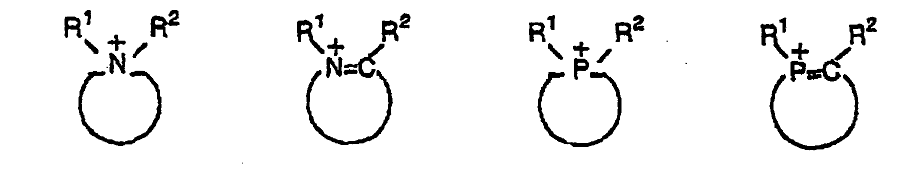
bei denen R¹,R²,R³,R⁴, identisch oder verschieden Wasserstoff, ohne NH₄⁺, und Hydrocarbylreste mit 1 bis 12 Kohlenstoffatomen darstellen und in denen die Ringe aus 4 bis 10 Kohlenstoffatomen bestehen.

5. Verfahren nach einem der Ansprüche 1 bis 3, **dadurch gekennzeichnet, dass** die quarternären Ammonium- und/oder Phosphoniumkationen als allgemeine Formeln haben:
**R**^{**1**}**R**^{**2**}**+N=CR**^{**3**}**-R**^{**5**}**-R**^{**3**}**C=N+R**^{**1**}**R**^{**2**}
**R**^{**1**}**R**^{**2**}**+P=CR**^{**3**}**-R**^{**5**}**-R**^{**3**}**C=P+R**^{**1**}**R**^{**2**}
bei denen R¹,R²,R³, identisch oder verschieden Wasserstoff oder Hydrocarbylreste mit 1 bis 12 Kohlenstoffatomen darstellen und R⁵ einen Alkylen- oder Phenylenrest darstellt.

6. Verfahren nach einem der vorhergehenden Ansprüche, **dadurch gekennzeichnet, dass** die quarternären Ammonium- und/oder Phosphoniumkationen aus der Gruppe gewählt sind, die gebildet wird durch N-Butylpyridinium, N-Ethylpyridinium, 3-Butyl-1-Methyl-lmidazolium, Diethylpyrazolinium, 3-Ethyl-1-Methyl-Imidazoliumin, Pyridinium, Trimethylphenylammonium, Tetrabutylphosphonium.

7. Verfahren nach einem der vorhergehenden Ansprüche, **dadurch gekennzeichnet, dass** die quarternären Ammonium- und/oder quarternären Phosphoniumsalze aus der Gruppe gewählt sind, die gebildet wird durch Hexafluorphosphat von N-Butylpyridinium, Tetrafluorborat vom N-Ethylpyridinium, Tetrafluorborat von Tetrabutylphosphonium, Hexafluorantimonat von 3-Butyl-1-Methyl-lmidazolium, Hexafluorphosphat von 3-Butyl-1-Methyl-Imidazolium, Triflourmethylsulfonat von 3-Butyl-1-Methyl-Imidazolium, Fluorsulfonat von Pyridinium, Hexafluorphosphat von Trimethylphenylammonium, Dichlorcuprat von 3-Ethyl-1-Methyl-lmidazoliumin, Tetrachlorbrorat von Pyridinium, Tetrachloraluminat von 3-Buthyl-1-Methyl-Imidazoliumin, Trichlorzinkat von 3-Buthyl-1-Methyl-Imidazoliumin.

8. Verfahren nach einem der vorhergehenden Ansprüche, **dadurch gekennzeichnet, dass** das Übergangsmetall Cobalt, Rhodium, Iridium, Ruthenium, Palladium und Platin ist.

9. Verfahren nach einem der vorhergehenden Ansprüche, **dadurch gekennzeichnet, dass** die Übergangsmetallverbindung ein Übergangsmetallkomplex ist.

10. Verfahren nach einem der vorhergehenden Ansprüche, **dadurch gekennzeichnet, dass** Übergangsmetallverbindung aus der Gruppe gewählt sind, die gebildet wird durch HRh(CO)(PR₃)₃, HRh(CO)₂(PR₃), HRh(CO)[P(OR₃)]₃, Rh(acac)(CO)₂, (wobei acac Acetylacetonat bedeutet) Rh₆(CO)₁₆, [Rh(CO)₃(PPh₃)₂]⁺BPh₄^{⁻}, RhCl(CO)(PEt₃)₂, [RhCl(Cyclooctadien)]₂, [Rh(CO)₃(PR₃)₂]⁺[BPh₄]⁻, [Rh(CO)₃(PR₃)₂]⁺BF₆^{⁻}, [Rh(Norbornadien)(PR₃)₂]⁺[BF₆]⁻, HCo(CO)₄, Ru₃(CO)₁₂, [RuH(CO)(Acetonitril)₂(PPh₃)₃]⁺[BF₄]⁻, PtCl₂(Cyclooctadien), [Ir(CO)₃(PPh₃)₃]⁺ [PF₆]⁻, [HPt(PEt₃)₃]⁺[PF₆]⁻, Rh₂O₃, Pd₂(NO₃)₂ und Rh(NO₃)₃.

11. Verfahren nach einem der Ansprüche 1 bis 10, **dadurch gekennzeichnet, dass** das Phosphinoxid wenigstens eine Amin-, Ammonium-, Alkohol-, Carboxylsäure-, oder Sulfonatfunktion enthält.

12. Verfahren nach einem der vorhergehenden Ansprüche, **dadurch gekennzeichnet, dass** die Konzentration der Verbindung oder der Verbindungen des Übergangsmetalls oder der Übergangsmetalle im Verhältnis zu dem Ammonium- und/oder Phosphoniumsalz 1 bis 500 mmol pro Liter ist.

13. Verfahren nach einem der vorhergehenden Ansprüche, **dadurch gekennzeichnet, dass** die katalytische Zusammensetzung ebenfalls ein organisches Lösungsmittel enthält.

14. Verfahren nach Anspruch 13, **dadurch gekennzeichnet, dass** das Lösungsmittel aus der Gruppe gewählt wird, die gebildet wird durch die aromatischen Kolenwasserstoffe und die aliphatischen Kohlenwasserstoffe.

15. Verfahren nach einem der Ansprüche 1 bis 14, **dadurch gekennzeichnet, dass** die organische, die Aldehyde enthaltende, Phase von der polaren Phase getrennt wird, wobei die polare Phase, die wenigstens einen Teil des Katalysators enthält, wenigstens zum Teil in den Reaktor zur Hydroformylierung rezykliert wird.

16. Verfahren nach einem der Ansprüche 1 bis 15, das zwischen 30 und 200°C unter einem Gesamtdruck zwischen 1 MPa und 20 MPa arbeitet, wobei die Verhältnisse der Partialdrucke von Kohlendioxid zu Wasserstoff 1:10 bis 10:1 sind.
